# EUROPEAN PATENT APPLICATION

(11) **EP 1 839 674 A1**
(43) Date of publication of application: **03.10.2007**
(21) Application number: 07008815.8
(22) Date of filing: 02.10.2000
(51) Int. Cl.: A61K 39/395, A61P 17/06, C07K 16/28

(54) **CD40 antagonist for treating psoriasis**

(30) Priority: 04.10.1999 US 157461 P
(62) Divisional of application: 00972003.8
(71) Applicant: Novartis Vaccines and Diagnostics, Inc., Emeryville, CA 94608-2916 (US)
(72) Inventor: Chu, Keting, Emeryville, CA 94608 (US); Wang, Changyu, Emeryville, CA 94608 (US)
(74) Representative: UEXKÜLL & STOLBERG

(57) **Abstract**

CD40 antagonists are used to prepare compositions, including pharmaceutical compositions, for treating autoimmune and neoplastic diseases in a mammal. The CD40 antagonist compositions are useful for reversing or substantially diminishing such autoimmune diseases as systemic lupus erythematosus, rheumatoid arthritis, multiple sclerosis and psoriasis.

## Description

### TECHNICAL AREA OF THE INVENTION

This invention relates to compositions for and methods of treating autoimmune and neoplastic diseases by administering one or more CD40 antagonist to a mammal.

### BACKGROUND OF THE INVENTION

Psoriasis is one of the most prevalent, yet enigmatic, chronic, inflammatory skin disorders in humans, afflicting approximately 2% of the population. Despite intensive efforts to develop treatments, this autoimmune disease remains substantially refractory to therapy. Thus, there remains a critical need to identify new agents and methods for the treatment of psoriasis and other related autoimmune diseases. The compositions and methods of the present invention fulfill these and other related needs.

### SUMMARY OF THE INVENTION

The present invention provides, in one embodiment, compositions, including pharmaceutical compositions thereof, that comprise a therapeutically effective amount of a CD40 antagonist. By the present invention, CD40 antagonists may be monoclonal or polyclonal antibodies, including humanized or human antibodies. Alternatively, inventive CD40 antagonists include suitable proteins or peptides or other small molecules that bind to CD40 thereby inhibiting the interaction of CD40 with its ligand (CD40L). The CD40 antagonist compositions can be formulated in amounts sufficient to reverse or diminish the severity of one or more autoimmune diseases including psoriasis. Inventive compositions may further comprise a pharmaceutically acceptable carrier or stabilizer suitable for *in vivo* administration. In some embodiments, these compositions may be further combined with additional agents efficacious against autoimmune diseases.

In other embodiments, the present invention provides methods for treating autoimmune diseases, which methods comprise the administration of a CD40 antagonist and pharmaceutical compositions thereof. More specifically, an amount of an inventive composition sufficient to inhibit or prevent an autoimmune disease is administered to and thereby contacted with the CD40 expressing cells. Autoimmune diseases encompassed within the scope of the instant methods include, but are not limited to, Hashimoto's thyroiditis, primary myxoedema thyrotoxicosis, pernicious anemia, Addison's disease, insulin-dependent diabetes mellitus, systemic lupus erythematosus (SLE), rheumatoid arthritis (RA), multiple sclerosis, dermatomyositis, scleroderma and psoriasis. The CD40 antagonist may be administered in a variety of ways including orally, topically and parenterally.

Further embodiments provide methods for treating a neoplastic disease. More specifically, by some embodiments, an amount of an inventive composition sufficient to reduce angiogenesis is administered to and contacted with CD40 expressing cells thereby reducing the severity of or reversing altogether the neoplastic disease.

In still further embodiments of the present invention, the methods may be performed either *ex vivo* or *in vitro.* For example, a CD40 antagonist may be applied to peripheral blood mononuclear cells (PBMC) isolated from a subject in need of anti-autoimmune disease therapy prior to reintroducing the PBMC *in vivo.* Alternatively, the present invention provides that CD40 antagonists may find use *in vitro* in, for example, diagnostic assays for the efficacy of other potential autoimmune disease therapeutics.

The present invention thus provides the art with compositions and methods which are generally effective in treating autoimmune diseases and, more specifically, in treating inflammatory skin diseases as exemplified by psoriasis.

### DETAILED DESCRIPTION OF THE INVENTION

Psoriasis is a T-cell mediated autoimmune disease believed to be linked to both genetic and environmental triggering factors such as bacterial superantigens. *See, e.g.,* Valdimarsson, H. et al., Immunol. Today, 16(3):145-9 (March 1995); Boehncke, W.H. et al., Nature, 379(6568):777 (February 29, 1996); Boehncke, W.H., Trends Microbiol., 4(12):485-9 (Dec. 1996). This disease is characterized by complex alterations of various cell types including parakeratosis, the hyperproliferation and differentiation of the epidermal keratinocytes, and akanthosis, the increase in epidermal thickness resulting from keratinocyte hyperproliferation. In addition, psoriatic lesions exhibit an infiltration of mixed leukocytes composed of activated T lymphocytes, neutrophils within the dermis and epidermal microabscesses, lining macrophages and dermal mast cells. Schon, M.P., J. Invest. Derm., 112(4):405-410 (1999).

CD40 is a 40-50 kDa type I membrane glycoprotein belonging to the TNF-R family and constitutively expressed on B lymphocytes as well as on monocytes, dendritic cells, endothelial cells and epithelial cells. *See* van Kooten, C. et al., Int. Arch. Allergy Immunol., 113:393-399 (1997); Datta, S.K. et al., Arthritis Rheum, 40(10):1735-45 (1997). The CD40 ligand, referred to variously as CD40L, gp39 or CD154, is a 33 kDa type II membrane glycoprotein that is transiently expressed primarily on the surface of activated CD4⁺ T cells. Datta, *supra.*

It has been discovered, as part of the present invention, that CD40 antagonists diminish the severity of autoimmune disease in an animal model system for psoriasis. Thus, the present invention provides compositions and methods for treating autoimmune diseases in an afflicted mammal which compositions comprise a CD40 antagonist and which methods comprise the administration of compositions comprising CD40 antagonists. It has also been discovered that inventive CD40 antagonists reduce the extent of angiogenesis in treated lesions. This discovery suggests the efficacy of CD40 antagonists in the treatment of various neoplastic diseases.

As used herein, the term "antagonist" generally refers to the property of a molecule, compound or other agent to, for example, interfere with the binding of one molecule with another molecule or the stimulation of one cell by another cell either through steric hindrance, conformational alterations or other biochemical mechanism. In one regard, the term antagonist relates to the property of an agent to prevent the binding of a receptor to its ligand, e.g., the binding of CD40 with CD40L, thereby inhibiting the activation of the respective B- or T-cell population. The term antagonist is not limited by any specific action mechanism, but, rather, refers generally to the functional property presently defined. Antagonists of the present invention include, but are not limited to, antibodies or peptides as well as other molecules that bind to CD40.

Effective therapeutics depend on identifying efficacious agents devoid of significant toxicity. Compounds potentially useful in treating psoriasis and other autoimmune diseases may be screened in a number of systems. Animal models are used to identify those compounds having therapeutic activity *in vivo* as well as possessing acceptable levels of host toxicity. The models preferably assess characteristics of psoriasis such as akanthosis and parakeratosis as well as inflammatory lymphocyte infiltration. Alternatively, animal models are also useful for identifying compounds that are efficacious in the treatment of other autoimmune diseases such as, *e.g.*, systemic lupus erythematosus, rheumatoid arthritis and multiple sclerosis or against various neoplastic diseases.

Efficacy of a given CD40 antagonist can be tested in any of the animal model systems familiar to those skilled in the art. Animal model systems for autoimmune diseases are described in Roitt, I. et al., "Autoimmunity and Autoimmune Disease," Immunology, Ch. 28 (1998); animal model systems available for the study of psoriasis, in particular, are described in Schon, M.P., *supra.* The skilled artisan will appreciate that the selection of an appropriate animal model system will depend on the particular disease being treated. The following animal model systems are, therefore, provided by way of example not limitation.

It is well known in the art that autoimmunity can be induced in experimental animals by injecting autoantigen (*i.e.*, self antigen) together with Freund's adjuvant. Thus, such an animal model system may be used, for example, by injecting thyroglobulin to induce an inflammatory disease of the thyroid. With such a model system, not only are thyroid autoantibodies produced, but, also, the gland becomes infiltrated with mononuclear cells and the acinar architecture deteriorates. This animal model has been used to model the human condition known as Hashimoto's thyroiditis. In a similar fashion, myelin basic protein, or T-cells specific for myelin basic protein, may be injected in mice or rats to induce autoallergic encephalomyelitis.

Alternative animal model systems that may be used to test compounds and treatment regimens within the scope of the present invention include animals exhibiting spontaneous autoimmune diseases. By way of example and not limitation, the Obese strain (OS) of chicken is characterized by the spontaneous occurrence of autoantibodies and by the progressive destruction and chronic inflammation of the thyroid. The OS chicken parallels human autoimmune thyroid disease in displaying thyroid lesions as well as the production of antibodies to various thyroid components.

A number of animal model systems for psoriasis have been described including transplantation of human psoriatic skin onto nude mice, the asebia (*ab*/*ab*) strain of mice or the HLA-B27 transgenic rat as well as transplantation of skin from the flaky skin mouse onto nude mice. Nickoloff, B.J. et al., Am. J. Path., 146(3):580-588 (1995); Schon, *supra.* The asebia mouse model features epidermal akanthosis, increased dermal vascularity and dermal infiltrate of macrophages and mast cells, but does not contain T-cell and neutrophil infiltrates. Nickoloff, *supra.* Thus, the skin alterations in the *ab*/*ab* mouse do not precisely mirror every biological characteristic of psoriatic lesions.

In addition to the above mentioned animal model systems, the SCID mouse is widely used as an *in vivo* model of psoriasis. A standard measure of efficacy in the SCID model is the ability to lessen the severity of akanthosis and parakeratosis as well as to reduce mononuclear cell infiltrate in animals transplanted with psoriatic skin. In the experiments described herein, the antibody preparations substantially inhibit the severity of psoriasis in animals. These findings indicate that symptoms of psoriasis can be inhibited or completely prevented by administration of antibodies or other substances having antagonistic effects on CD40.

In recent years, it has been observed that human skin cells can be engrafted onto severe combined immunodeficiency (SCID) mice with long-term graft survival. The SCID mouse is also amenable to the adoptive transfer of components of the human immune system. *See, e.g.,* Boehncke, W.-H. et al., Arch. Dermatol. Res., 286:325-330 (1994). The autosomal recessive mutation responsible for the SCID phenotype in mice prevents antigen receptor gene rearrangements resulting in an intrinsic defect of T- and B-cells. Botsma, M.J. et al., Annu. Rev. Immunol., 9:323-350 (1991). Nickoloff, *supra,* reported that psoriatic plaque skin (PP), normal human skin from healthy individuals (NN) and symptomless skin from a patient with psoriasis (PN) can be transplanted onto SCID mice with retention of clinical, histological and immunological phenotypic characteristics.

The various animal models for psoriasis have been reviewed by M.P. Schon, *supra.* Schon reported that the SCID mouse xenogeneic skin transplant model system exhibits the morphological and pathological characteristics of naturally occurring human psoriasis. For example, psoriatic human skin transplanted onto the SCID mouse maintains the psoriatic phenotype as evidenced by akanthosis and hyperproliferation. Also, transplanted skin is characterized by altered keratinocyte differentiation, induction of MHC Class II and ICAM-1, increased vascularity, T-cell and neutrophil infiltrate and intraepidermal microabscesses. Thus, Schon endorses the SCID mouse for studies of antipsoriatic treatments noting, in particular, that the attractiveness of this animal model stems from its reliance on actual human tissue.

With the SCID mouse xenogeneic transplantation model, investigators have studied the relative contributions of various components of the immune system to the etiology and pathophysiology of psoriasis. Nickoloff, *supra,* reported the validity of the SCID mouse animal model system in 1995 and disclosed its utility in studies designed to decipher the mechanism underlying the genetic and etiological abnormalities associated with psoriasis as well as to illuminate the disease's pathophysiological basis. *Id.* Wrone-Smith, T. et al., further demonstrated the utility of the SCID animal model system in mechanistic studies from which it was reported that psoriasis is mediated by immunocytes derived from the circulation and that activated immunocompetent cells secondarily induce keratinocyte and endothelial cell proliferation. J. Clin. Invest., 98(8):1878-1887 (1996). More recently, Gilhar, A. et al. investigated the role of T lymphocytes in psoriatic pathology using the SCID mouse animal model system, J. Invest. Derm., 109(3):283-288 (1997), noting that skin-infiltrating T lymphocytes, but not T-cells derived from peripheral blood, maintained the psoriatic phenotype of human skin grafted onto SCID mice. And, most recently, Torres, B.A. et al. used the SCID mouse model to study the role of bacterial and viral superantigens in the progression of psoriasis. Cur. Opin. Immunol., 10(4):465-470 (1998).

The CD40 antagonists may inhibit the up-regulation of activation markers, *e.g.*, CD25 and CD69, on CD4⁺ T-cells to between about 10 and 30% the levels of the untreated control cells. In addition, inventive CD40 antagonists are effective in inhibiting the morphological characteristics of psoriasis such as epidermal thickening and hyperproliferation, *i.e.*, akanthosis and parakeratosis, respectively, in the SCID mouse xenogeneic transplant animal model system. Furthermore, administration of the presently disclosed CD40 antagonists to SCID mice transplanted with psoriatic skin grafts substantially reduced the extent of mononuclear infiltrate in the upper dermis of these mice. These findings document that administration of CD40 antagonists generally is effective in the treatment of established lesions from chronic plaque-stage psoriasis. More particularly, the present invention demonstrates that the CD40 antagonist antibody 5H7 is efficacious in the treatment of psoriasis.

Inventive CD40 antagonists also reduce the extent of angiogenesis in the SCID mouse xenogeneic transplant model system suggesting that these molecules may be efficacious in the treatment of neoplastic disease.

The CD40 antagonists described herein can be used to treat other autoimmune diseases characterized by interaction of CD40 with its ligand CD40L. As used herein, the phrase "autoimmune disease" refers generally to those diseases characterized by the failure of one or more B- and/or T-cell populations, or gene products thereof, to distinguish between self and non-self antigenic determinants. Autoimmune diseases are often characterized by the infiltration of the target cells with inflammatory lymphoid cells, for example, mononuclear phagocytes, lymphocytes and plasma cells as well as secondary lymphoid follicles. Exemplary autoimmune diseases include, but are not limited to, organ specific disorders such as Hashimoto's thyroiditis, primary myxoedema thyrotoxicosis, pernicious anemia, Addison's disease, and insulin-dependent diabetes mellitus as well as non-organ specific disorders such as systemic lupus erythematosus (SLE), rheumatoid arthritis (RA), multiple sclerosis, dermatomyositis, scleroderma and psoriasis.

As provided herein, the compositions for and methods of treating autoimmune diseases may utilize one or more antibody used singularly or in combination with other therapeutics to achieve the desired diminution of the autoimmune disease of interest. Antibodies according to the present invention may be isolated from an animal producing the antibody as a result of either direct contact with an environmental antigen or immunization with the antigen. Alternatively, antibodies may be produced by recombinant DNA methodology using one of the antibody expression systems well known in the art. *See, e.g.,* Harlow and Lane, Antibodies: A Laboratory Manual, Cold Spring Harbor Laboratory (1988). Such antibodies may include recombinant IgGs, chimeric fusion proteins having immunoglobulin derived sequences or "humanized" antibodies that may all be used for the treatment of autoimmune diseases according to the present invention. In addition to intact, full-length molecules, the term antibody also refers to fragments thereof (such as, *e.g.*, scFv, Fv, Fd, Fab, Fab' and F(ab)'₂ fragments) or multimers or aggregates of intact molecules and/or fragments that bind to CD40. These antibody fragments bind antigen and may be derivatized to exhibit structural features that facilitate clearance and uptake, *e.g.*, by incorporation of galactose residues.

In one embodiment of the present invention, CD40 antagonists are monoclonal antibodies prepared essentially as described in de Boer et al. US Patent No. 5,677,165 (1997) (de Boer '165) which patent is incorporated by reference herein. By this method, DNA encoding CD40 or a fragment thereof is PCR amplified from a mixture of cellular cDNAs. The PCR product is digested with one or more restriction endonucleases to create appropriate ends and ligated into a baculovirus plasmid or other expression system. In the case of a baculovirus expression system, the plasmid encoding CD40, or a fragment thereof, is introduced into, *e.g.*, Sf9 cells to facilitate protein production. Clones of Sf9 cells expressing CD40 are identified, *e.g.*, by ELISA as discussed in de Boer '165 and injected, intraperitoneally, into BALB/c mice to induce antibody production. Serum is tested for the production of specific antibodies and spleen cells from animals having a positive specific antibody titer are used for cell fusions with myeloma cells to generate hybridoma clones. Supernatants derived from hybridoma clones are tested, via fluorescent cell staining of EBV-transformed B-cells, for the presence of monoclonal antibodies having specificity against CD40.

In other embodiments of the present invention, CD40 antagonists are humanized anti-CD40 monoclonal antibodies. The phrase "humanized antibody" refers to an antibody derived from a non-human antibody - typically a mouse monoclonal antibody. Alternatively, a humanized antibody may be derived from a chimeric antibody that retains or substantially retains the antigen-binding properties of the parental, non-human, antibody but which exhibits diminished immunogenicity as compared to the parental antibody when administered to humans. The phrase "chimeric antibody," as used herein, refers to an antibody;containing sequences derived from two different antibodies (*see, e.g.,* U.S. Patent no. 4,816,567), which typically originate from different species. Most typically, chimeric antibodies comprise human and murine antibody fragments, generally human constant and mouse variable regions.

Humanized antibodies may be achieved by a variety of methods including, for example: (1) using the non-human complementarity determining regions (CDRs) with a human framework and constant region (a process referred to in the art as "humanizing"), or, alternatively, (2) transplanting the entire non-human variable domains, but "cloaking" them with a human-like surface by replacement of surface residues (a process referred to in the art as "veneering"). In the present invention, humanized antibodies will include both "humanized" and "veneered" antibodies. These methods are disclosed, *e.g.,* in Jones et al., Nature 321:522-525 (1986); Morrison et al., Proc. Natl. Acad. Sci., U.S.A., 81:6851-6855 (1984); Morrison and Oi, Adv. Immunol., 44:65-92 (1988); Verhoeyer et al., Science 239:1534-1536 (1988); Padlan, Molec. Immun. 28:489-498 (1991); and Padlan. Molec. Immunol. 31(3):169-217 (1994) (each of these methods is incorporated herein by reference).

The phrase "complementarity determining region" refers to amino acid sequences which together define the binding affinity and specificity of the natural Fv region of a native immunoglobulin binding site. *See, e.g.,* Chothia, et al., J. Mol. Biol. 196:901-917 (1987); Kabat et al., U.S. Dept. of Health and Human Services NIH Publication No. 91-3242 (1991). The phrase "constant region" refers to the portion of the antibody molecule which confers effector functions. In one embodiment of the antibodies of the present invention, mouse constant regions are substituted by human constant regions. The constant regions of the subject humanized antibodies are derived from human immunoglobulins. The heavy chain constant region can be selected from any of the five isotypes: alpha, delta, epsilon, gamma or mu.

One method of humanizing antibodies comprises aligning the non-human heavy and light chain sequences to human heavy and light chain sequences, selecting and replacing the non-human framework with a human framework based on such alignment, molecular modeling to predict the conformation of the humanized sequence and comparing to the conformation of the parent antibody. This process is followed by repeated back mutation of residues in the CDR region which disturb the structure of the CDRs until the predicted conformation of the humanized sequence model closely approximates the conformation of the non-human CDRs of the parent non-human antibody. Such humanized antibodies may be further derivatized to facilitate uptake and clearance, *e.g.*, via Ashwell receptors, or other receptor mediated clearance mechanisms such as by the incorporation of galactose residues or other hexoses. *See, e.g.,* U.S. Patent Nos. 5,530,101 and 5,585,089 which patents are incorporated herein by reference.

Alternatively, humanized antibodies may be prepared essentially as described in de Boer, U.S. Patent No. 5,874,082 (1999) (de Boer '082) which patent is incorporated herein by reference. Briefly, mRNA is prepared from a hybridoma which expresses an anti-CD40 monoclonal antibody. cDNA encoding the variable regions of the heavy and light chains is amplified using RT-PCR employing degenerate oligonucleotide primers. As disclosed in de Boer '082, the RT-PCR technique is well known in the art and is incorporated herein by reference to Myers et al., Biochemistry, 30:7661-7666 (1991) and U.S. Patent Nos. 5,310,652 and 5,407,800. PCR products are cloned into a sequencing plasmid from which clones the nucleotide sequence of the variable heavy and light chain cDNAs are determined and from which sequence a consensus amino acid sequence for the variable heavy and light chains is derived.

The deduced amino acid sequences are used to search databases for human antibody sequences having the highest degree of sequence similarity to the monoclonal antibody (de Boer '082). Based on the identified homologous human sequence, mutagenesis primers are designed and used to change the indicated residues from mouse to human. cDNAs encoding the humanized variable heavy and light chains are expressed off a baculovirus expression plasmid including a portion of the constant region of human IgG heavy chain and the complete human constant light chain. Humanized heavy and light chains are co-expressed in Sf9 insect cells and the resulting culture supernatants are analyzed for antibody expression using Western blot and fluorescence-activated cell sorting (FACS) analysis (de Boer '082).

The monoclonal antibodies of the invention can also be produced using transgenic animals that are engineered to contain human immunoglobulin loci. For example, WO 98/24893 discloses transgenic animals having a human Ig locus wherein the animals do not produce functional endogenous immunoglobulins, due to the inactivation of endogenous heavy and light chain loci. WO 91/10741 also discloses transgenic non-primate mammalian hosts capable of mounting an immune response to an immunogen, wherein the antibodies have primate constant and/or variable regions, and wherein the endogenous immunoglobulin-encoding loci are substituted or inactivated. WO 96/30498 discloses the use of the Cre/Lox system to modify the immunoglobulin locus in a mammal, such as to replace all or a portion of the constant or variable region to form a modified antibody molecule. WO 94/02602 discloses non-human mammalian hosts having inactivated endogenous Ig loci and functional human Ig loci. U.S. Patent No. 5,939,598 discloses methods of making transgenic mice in which the mice lack endogenous heavy claims, and express an exogenous immunoglobulin locus comprising one or more xenogeneic constant regions.

Using a transgenic animal described above, an immune response can be produced to a selected antigenic molecule, in this case CD40, and antibody-producing cells can be removed from the animal and used to produce hybridomas that secrete human monoclonal antibodies. Immunization protocols, adjuvants, and the like are known in the art, and are used in immunization of, for example, a transgenic mouse as described in WO 96/33735. The monoclonal antibodies can be tested for the ability to inhibit or neutralize the biological activity or physiological effect of the corresponding protein.

Examples of suitable human anti-CD40 monoclonal antibodies are disclosed in applicants' co-pending application entitled "Human Anti-CD40 Monoclonal Antibodies," filed October 2, 2000, as Serial No. which is incorporated herein by reference. The application discloses CD40 antibodies raised in mice transgenic for human immunoglobulin loci. The antibodies specifically bind to CD40 expressed by a variety of cells, and are suitable for use in the methods of the present invention.

The CD40 antagonists of the present invention are said to be immunospecific or specifically binding if they bind to CD40 with a Kₐ of greater than or equal to about 10⁴ M⁻¹, preferably of greater than or equal to about 10⁵ M⁻¹, more preferably of greater than or equal to about 10⁶ M⁻¹ and still more preferably of greater than or equal to about 10⁷ M⁻¹. Such affinities may be readily determined using conventional techniques, such as by equilibrium dialysis; by using the BIAcore 2000 instrument, using general procedures outlined by the manufacturer; by radioimmunoassay using ¹²⁵I-labeled CD40; or by another method known to the skilled artisan. The affinity data may be analyzed, for example, by the method of Scatchard et al., Ann N.Y. Acad. Sci., 51:660 (1949). Thus, it will be apparent that preferred CD40 antagonists will exhibit a high degree of specificity for CD40 and will bind with substantially lower affinity to other molecules.

Identification of additional CD40 antagonists may be achieved by using any of a number of known methods for identifying and obtaining proteins that specifically interact with other proteins or polypeptides, for example, a yeast two-hybrid screening system such as that described in U.S. Patent No. 5,283,173 and U.S. Patent No. 5,468,614, or the equivalent may be utilized. In one embodiment of the present invention, a cDNA encoding CD40, or a fragment thereof, may be cloned into a two-hybrid bait vector and used to screen a complementary target library for a protein having CD40 binding activity.

As used herein, the term "protein" includes proteins, oligopeptides, polypeptides, peptides and the like. Additionally, the term protein may also refer to fragments, multimers or aggregates of intact molecules and/or fragments. Proteins may be naturally occurring or may be produced via recombinant DNA means or by chemical and/or enzymatic synthesis. *See, e.g.,* Sambrook et al., Molecular Cloning: A Laboratory Manual, Cold Spring Harbor Laboratories (2nd ed. 1989).

In addition to antibodies and other proteins, this invention also contemplates alternative CD40 antagonists including, but not limited to, small molecules that are also effective in treating various autoimmune and/or neoplastic diseases. Such small molecules may be identified by assaying their capacity to bind to CD40 and/or to inhibit the interaction between CD40 and CD40L.

Methods for measuring the binding of CD40 with small molecules are readily available in the art and include, for example, competition assays whereby the small molecule interferes with the interaction between CD40 and its ligand (CD40L) or an anti-CD40 antibody. Alternatively, direct binding assays may be utilized to measure the interaction of a small molecule with CD40. By way of example, an ELISA assay may be employed whereby CD40, or a CD40 extracellular domain, is adsorbed onto an insoluble matrix such as a tissue culture plate or bead. A labeled CD40L or anti-CD40 antibody is blocked from binding to CD40 by inclusion of the small molecule of interest. Alternatively, the binding of a small molecule to CD40 may be determined by a fluorescence activated cell sorting (FACS) assay. By this method, cells expressing CD40 are incubated with a fluorescent tagged anti-CD40 antibody or an anti-CD40 antibody in the presence of a fluorescent tagged secondary antibody. Binding of a small molecule to CD40 may be assessed by a dose dependent decrease in fluorescence bound to the CD40 expressing cells. Similarly, direct binding of a small molecule may be assessed by labeling, *e.g.* radiolabeling or fluorescent tagging, the small molecule, incubating with immobilized CD40 or CD40 expressing cells and assaying for the radioactivity or fluorescence of the bound small molecule.

CD40 antagonists of the present invention include, where applicable, functional equivalents. For example, molecules may differ in length, structure, components, etc. but may still retain one or more of the defined functions. More particularly, functional equivalents of the antibodies, antibody fragments or peptides of the present invention may include mimetic compounds, *i.e.*, constructs designed to mimic the proper configuration and/or orientation for antigen binding.

Preferred CD40 antagonists may optionally be modified by addition of side groups, etc., *e.g.*, by amino terminal acylation, carboxy terminal amidation or by coupling of additional groups to amino acid side chains. Antagonists may also comprise one or more conservative amino acid substitutions. By "conservative amino acid substitutions" is meant those changes in amino acid sequence that preserve the general charge, hydrophobicity/hydrophilicity and/or steric bulk of the amino acid substituted. For example, substitutions between the following groups are conservative: Gly/Ala, Val/Ile/Leu, Asp/Glu, Lys/Arg, Asn/Gln, Ser/Cys,/Thr, and Phe/Trp/Tyr. Such modifications will not substantially diminish the efficacy of the CD40 antagonists and may impart such desired properties as, for example, increased *in vivo* half-life or decreased toxicity.

Having identified more than one CD40 antagonist that is effective in an animal model, it may be further advantageous to mix two or more such CD40 antagonists together to provide still improved efficacy against autoimmune diseases. Compositions comprising one or more CD40 antagonist may be administered to persons or mammals suffering from, or predisposed to suffer from, an autoimmune disease. CD40 antagonists are believed to minimize the severity of autoimmune diseases by reducing the infiltration of target cells with inflammatory lymphoid cells such as mononuclear phagocytes, lymphocytes, plasma cells and secondary lymphoid follicles and, in the specific case of psoriasis, by diminishing the severity of akanthosis and parakeratosis.

By the present methods, compositions comprising CD40 antagonists may be administered parenterally, topically, orally or locally for therapeutic treatment. Preferably, the compositions are administered orally or parenterally, *i.e.,* intravenously, intraperitoneally, intradermally or intramuscularly. Thus, this invention provides methods which employ compositions for administration which comprise one or more CD40 antagonists in a pharmaceutically acceptable carrier, preferably an aqueous carrier. A variety of aqueous carriers may be used, *e.g.*, water, buffered water, 0.4% saline, 0.3% glycine and the like, and may include other proteins for enhanced stability, such as albumin, lipoprotein, globulin, etc., subjected to mild chemical modifications or the like.

CD40 antagonists useful as therapeutics for autoimmune diseases will often be prepared substantially free of other naturally occurring immunoglobulins or other biological molecules. Preferred CD40 antagonists will also exhibit minimal toxicity when administered to a mammal afflicted with an autoimmune disease.

The compositions of the invention may be sterilized by conventional, well known sterilization techniques. The resulting solutions may be packaged for use or filtered under aseptic conditions and lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride and stabilizers (*e.g.*, 1-20% maltose, etc.).

The CD40 antagonists of the present invention may also be administered via liposomes. Liposomes, which include emulsions, foams, micelles, insoluble monolayers, phospholipid dispersions, lamellar layers and the like, can serve as vehicles to target the CD40 antagonists to a particular tissue as well as to increase the half-life of the composition. A variety of methods are available for preparing liposomes, as described in, *e.g.*, U.S. Patent Nos. 4,837,028 and 5,019,369, which patents are incorporated herein by reference.

The concentration of the CD40 antagonist in these compositions can vary widely, i.e., from less than about 10%, usually at least about 25% to as much as 75% or 90% by weight and will be selected primarily by fluid volumes, viscosities, etc., in accordance with the particular mode of administration selected and the autoimmune disease being treated. Actual methods for preparing orally, topically and parenterally administrable compositions will be known or apparent to those skilled in the art and are described in detail in, for example, Remington's Pharmaceutical Science, 19th ed., Mack Publishing Co., Easton, PA (1995), which is incorporated herein by reference.

Determination of an effective amount of a composition of the invention to treat an autoimmune disease in a patient can be accomplished through standard empirical methods which are well known in the art. For example, in the case of psoriasis, reversal of akanthosis and parakeratosis as well as diminution in lymphocyte infiltration in the keratinocytes can be measured.

Compositions of the invention are administered to a mammal already suffering from an autoimmune disease or predisposed to an autoimmune disease in an amount sufficient to prevent or at least partially arrest the development of the autoimmune disease. Similarly, inventive compositions may be administered to a mammal afflicted with a neoplastic disease in order to reduce the disease's severity. An amount adequate to accomplish this is defined as a "therapeutically effective dose." Effective amounts of a CD40 antagonist will vary and depend on the severity of the disease and the weight and general state of the patient being treated, but generally range from about 1.0 µg/kg to about 100 mg/kg body weight, with dosages of from about 20 µg/kg to about 10 mg/kg per application being more commonly used. Administration is daily, weekly or less frequently, as necessary depending on the response to the disease and the patient's tolerance of the therapy. Maintenance dosages over a prolonged period of time may be needed, and dosages may be adjusted as necessary.

Single or multiple administrations of the compositions can be carried out with the dose levels and pattern being selected by the treating physician. In any event, the formulations should provide a quantity of CD40 antagonist sufficient to effectively prevent or minimize the severity of the autoimmune disease. The compositions of the present invention may be administered alone or as an adjunct therapy in conjunction with other therapeutics well known in the art for the treatment of psoriasis or other autoimmune disease.

The methods of the invention can also be employed for *ex vivo* or extracorporeal therapy against autoimmune diseases by performing the therapeutic manipulations on peripheral blood mononuclear cells (PBMC) outside of the body. For example, PBMC may be removed from the subject, and treated with the inventive CD40 antagonist. These cells may be subsequently administered to the subject to block or substantially reduce the activation of CD4⁺ T-cells. By performing the administration of the CD40 antagonist outside of the subject's body, significantly higher concentrations of the CD40 antagonist may be employed than would be tolerated through *in vivo* administration. *Ex vivo* applications of the present methods may further comprise the administration of additional agents which together provide enhanced therapeutic activity against autoimmune diseases.

The compositions of the present invention also find use *in vitro.* For example, CD40 antagonists can be used in screening assays to assess the effective levels of therapeutics or other treatments for autoimmune or neoplastic diseases. In other embodiments, the present compositions may be used in the design or screening of various potential treatment modalities, such as methods for the treatment of psoriasis or other autoimmune disease. Thus, a diagnostic method for assessing the efficacy of, *e.g.*, autoimmune therapeutics is also provided by the present invention. Detecting changes *in vitro* which parallel the reversal of an autoimmune or neoplastic disease provides an indication of *in vivo* activity on the CD40 antagonist intended for treatment in accordance with the present invention.

The following experimental examples are offered by way of illustration not limitation.

### EXAMPLE 1

This example describes the use of humanized mouse monoclonal antibody 5H7 to treat psoriasis in the severe combined immunodeficiency (SCID) mouse xenogeneic transplant model system.

SCID is an autosomal recessive mutation in C.B-17 mice that causes a lack of antigen receptor gene rearrangements leading to an intrinsic defect of T- and B-cells. Boehncke, W.-H., et al., Arch. Dermatol. Res., 286(6):325-30 (1994). Nickoloff, *supra,* documented that normal (NN); non-lesional, pre-psoriatic (PN); and lesional, psoriatic (PP) skin can be transplanted onto SCID mice with high rates of survival (*i.e.*, >85%). After transplantation, normal and psoriatic skin retain their respective morphological characteristics while pre-psoriatic skin becomes somewhat thicker. This animal model has received substantial attention as a viable model for performing mechanistic-type studies designed to reveal the genetic/etiological as well as pathophysiological bases for psoriasis. Efficacy in the SCID mouse xenogeneic transplantation model is determined by measuring, *inter alia,* decreased epidermal thickening, normalization of keratinization,: reestablishment of a granular layer and a reduction in inflammatory infiltration.

Using the SCID mouse xenogeneic transplant model system, uninvolved, non-lesional (PN) and involved, lesional (PP) human skin from three patients suffering from chronic plaque-stage psoriasis was transplanted onto mice following the procedure of Boehncke, W.-H. et al., *Arch. Dermatol. Res., supra,* which reference is incorporated herein in its entirety. From each human donor, six grafts of PN skin and six grafts of PP skin were isolated and transplanted onto a total of 12 mice. Grafts were allowed to heal in for a period of 4 weeks before the mice were subjected, in groups of three, to one of the following treatment regimens: (1) Treatment Group - mice transplanted with lesional PP skin were treated, by intraperitoneal (i.p.) injections, with antibody 5H7 at a dosage of 20 mg/kg every other day for 2 weeks; (2) Treatment Control Group - mice transplanted with lesional PP skin were treated, by i.p. injections, with isotype antibody MsIgG₁; (3) Prevention Group - mice transplanted with non-lesional PN skin were treated, by intradermal (i.d.) injections, with 2 x 10⁶ peripheral blood mononuclear cells (PBMC) pre-activated *ex vivo,* in the presence of antibody 5H7, with staphylococcal superantigens following the method of Wrone-Smith, T. et al. J. Clin Invest., 98:1878-1887 (1996), which reference is incorporated herein in its entirety. Subsequent to the PBMC injections, the mice of group (3) were further treated by *in vivo* administration of antibody 5H7 as per the "Treatment Group;" and (4) Prevention Control Group - mice transplanted with non-lesional PN skin were treated with i.d. injections of 2 x 10⁶ PBMC pre-activated *ex vivo* with staphylococcal superantigens as in the "Prevention Group" followed by administration of antibody MsIgG₁. Four weeks after termination of the manipulations, the grafts were harvested, fixed in formaldehyde and used for morphological analyses based on routine hematoxylin and eosin H+E staining.

To ensure comparability between the groups, grafts were macroscopically evaluated immediately prior to initiating the manipulations and groups were sorted such that grafts with macroscopic erosions were segregated into a separate group. These preliminary evaluations revealed that the majority of skin grafts took without evidence of rejection.

### In vitro Administration of 5H7 Inhibits Superantigen Activation of PBMCs.

In order to assess the potential of 5H7 to block activation of PBMCs by *ex vivo* administration of bacterial superantigens, aliquots of the PBMCs injected into PN skin transplanted mice were analyzed by 2-color fluorescence activated cell sorting (FACS). The results, summarized in Table 1, show that, with the sole exception of CD25 expression in donor B, 5H7 inhibited the up-regulation of the activation markers CD25 and CD69 on CD4⁺ T-cells to between 10 and 30% the levels of the untreated control cells.

**Table 1: Effects of Superantigen Activation of PBMCs In vitro with or without 5H7. CD4+CD25+**

| Donor | Antibody | CD3+CD69+ | CD3+CD25+ | CD4+CD69+ | D3+CD25+ |
|---|---|---|---|---|---|
| R | MsIgG₁ | 38.4 | 13.2 | 30.5 | 17.0 |
| | 5H7 | 33.7 | 11.3 | 26.3 | 14.8 |
| | | | | | |
| B | MsIgG₁ | 29.6 | 27.3 | 25.5 | 16.0 |
| | 5H7 | 30.7 | 10.6 | 21.0 | 18.0 |
| | | | | | |
| W | MsIgG₁ | 21.7 | 23.9 | 22.9 | 22.3 |
| | 5H7 | 17.6 | 14.6 | 15.8 | 15.5 |

### Effects of 5H7 on Treatment of Psoriasis In vivo.

Mice transplanted with lesional PP psoriatic skin and treated with the isotype control antibody MsIgG₁ exhibited akanthosis characterized by an elevated epidermal thickness of approximately 190-340 µm resulting from persistent epidermal hyperproliferation. Moreover, mice transplanted with PP skin and treated with MsIgG₁ also exhibited parakeratosis characterized by the partial to complete absence of a granular layer indicating the persistence of an alteration in the keratinization process typical for psoriasis. Additionally, a dense mononuclear infiltrate was seen located in the upper dermis of these mice.

*In vivo* treatment of PP skin transplanted mice with 5H7 resulted in a measurable reduction of the epidermal thickness ranging from 30% in human donors W and B to 50% in human donor R. Such reduced epidermal thickness stemmed from a decrease in epidermal hyperproliferation. Normalization of the keratinization pattern was mirrored by reestablishment of a granular layer; occasionally the regular web-like structure of the corneal layer could be observed. In addition, the inflammatory infiltrate was reduced to around 50%.

These findings document that 5H7 administration is effective in the treatment of established lesions from chronic plaque-stage psoriasis in the SCID mouse model system.

### EXAMPLE 2

In a second set of experiments, mice with skin grafts as described in Example 1 were divided into four treatment groups:
1. Low dose 5D12 treatment group: Two weeks after the grafting, anti-CD40 AB was injected ip every two days for two weeks (six doses) at 0.5 mg/kg dose. Two to four weeks later, grafts were harvested.
2. High dose 5D12 treatment group: Two weeks after grafting, anti-CD40 AB was injected ip every two days for two weeks (six doses) at 5 mg/kg dose. Two to four weeks later, grafts were harvested.
3. 5C8 (anti-CD40L) treatment group: Two weeks after grafting, anti-CD40 AB was injected ip every two days for two weeks (six doses) at 0.5 mg/kg dose. Two to four weeks later, grafts were harvested.
4. Isotype antibody treatment control group: Three lesional grafts for control AB treatment as above for group 2.

The anti-CD40 antibody treatment resulted in reduction of epidermal thickness by 30%-50% and normalization of the keratinization pattern (web-like structure of the corneal layer, reconstitution of the granular layer) as well as a slight reduction of the inflammation judged by the density of the infiltrate (about 20%-40% reduction, no Munro's microabscesses). The anti-CD40L antibody yields results similar to the anti-CD40 antibody in this model. A blinded observer could not assign the histologies to the treatment protocols, except for the negative control.

### EXAMPLE 3

### Evaluation of Low and High Dose CD40 Antibody 5D12

This experiment was conducted, using the mouse model described in Exmple 1, to quantitate the anti-psoriatic effects of low doses of anti-CD40 antibody. The low-dose treatment with antibody 5D12 consisted of intraperitoneal injections of mice with 0.5 mg/kg 5D12 every other day for two weeks. High-dose treatment consisted of intraperitoneal injections of mice with 5 mg/kg 5D12 every other day for two weeks. For 5C8 treatment, intraperitoneal injections with 5 mg/kg 5D12 were made every other day for two weeks. The treatment control group consisted of mice injected intraperitoneally with isotype control antibody for two weeks.

Four weeks after the termination of the treatments, the grafts were harvested and either fixed in formaldehyde or snap-frozen. Fixed material was used for morphological analyses based on H+E staining, and the snap-frozen material used for additional immunohistochemical analyses. The results are described below.

Effects of isotype control. Lesional psoriatic skin treated with the isotype control antibody exhibited an epidermal thickness of 300-570 mm, depending upon the donor. The results are shown in Tables 2-7. This thickening, when compared to non-lesional epidermis or normal epidermis, is referred to as akanthosis, and is due to persisting hyperproliferation of the keratinocytes, a hallmark of lesional psoriatic epidermis. The thickened epidermis exhibited a pronounced elongation of the rete ridges, along with a narrowing of epidermis overlying the dermalpapillae, resulting in a wave-like epidermo-dennal border; this phenomenon is referred to as papillomatosis. Disturbed differrentiation of keratinocytes is also typical for psoriasis and results in partial or complete absence of a granular layer, a phenomenon known as parakeratosis.

Shadows of the keratinocyte nuclei could be observed in the corneal layer where they nromally would not be visible (dyskeratosis); the corneal layer itself lacked the typical web-like structure and instead looked compact. Additionally, a dense mononuclear infiltrate was seen located in the upper dermis. Although these features were more or less present in all control grafts, those grafts that were kept on the mice over a period of 10 weeks (in contrast to 8 weeks) exhibited a less typical appearance of lesional psoriatic skin. The characteristics described above were present in a less pronounced fashion (e.g., the granular layer was more completely restored, stretches of web-like corneal layer occurred, and patches of dyskeratosis were less frequent). These results suggest that despie the relatively stable phenotype, there is some fading of the characteristics of psoriasis over time, possibly indicating a remission. This would parallel the natural course of the disease, which is characterized by a chronic-recurrent course.

Effects of treatment with antibody 5D12. Treatment with the anti-CD40 antibody 5D12 at a dose of 5 mg/kg (high dose) resulted in a reduction of the epidermal thickness ranging from 20% to 50% depending on the donor (Tables 2-4). Normalization of the keratinization pattern was mirrored by re-establishment of a granular layer, and occasionally the regular web-like structure of the corneal layer could be observed. Dyskeratotic patches could not be observed. Additionally, the inflammatory infiltrate was markedly reduced.

When grafts that had received the 5D12 antibody in a dose of 0.5 mg/kg (low dose) were compared to those treated with high dose 5D12, a blinded observer could not distinguish these groups, indicating that the histological characteristics looked similar. Quantification of the epidermal thickness documents effects comparable to the high dose group (reduction of 20-40%, Tables 2-4). However, in a side by side comparison, the effects appeared more pronounced in the high dose group, specifically, longer stretches of web-like corneal layer, and less dyskeratosis.

Effects of treatment with antibody 5C8. The antipsoriatic effects of the anti-CD40 ligand antibody 5C8 were analyzed at a dose of 5 mg/ml. As in the case of the high and low dose 5D12 treatment, considerable reduction of epidermal thickness, along with partial restoration of normal epidermal histology, could be induced. No clear difference between this group and the grafts receiving one of the other two treatment regimens could be reproducibly defined by a blinded observer (Tables 2-4).

These results confirm and extend the results shown in Examples 1 and 2 above, in which an anti-CD40 antibody exhibits measurable anti-psoriatic effects in the SCID-hu xenogeneic transplantation model.

**Table 2**

| Effects of High and Low Dose 5D12 | | | |
|---|---|---|---|
| **Graft number** | **Donor** | **Treatment** | **Epidermal thickness (mm)** |
| 60 | LS | Isotype control | 380 |
| 61 | LS | Isotype control | 340 |
| 62 | LS | Isotype control | 340 |
| 63 | LS | Low dose 5D12 | 290 |
| 64 | LS | Low dose 5D12 | 270 |
| 65 | LS | Low dose 5D12 | 270 |
| 66 | LS | High dose 5D12 | 290 |
| 67 | LS | High dose 5D12 | 210 |
| 68 | LS | High dose 5D12 | 250 |
| 69 | LS | 5C8 | 270 |
| 70 | LS | 5C8 | 300 |
| 71 | LS | 5C8 | 250 |

**Table 3**

| Effects of High and Low Dose 5D12 | | | |
|---|---|---|---|
| **Graft number** | **Donor** | **Treatment** | **Epidermal thickness (mm)** |
| 72 | BA | Isotype control | 570 |
| 73 | BA | Isotype control | 420 |
| 74 | BA | Isotype control | 490 |
| 75 | BA | Low dose 5D12 | 380 |
| 76 | BA | Low dose 5D12 | 380 |
| 77 | BA | Low dose 5D12 | 420 |
| 78 | BA | High dose 5D12 | 300 |
| 79 | BA | High dose 5D12 | 340 |
| 80 | BA | High dose 5D12 | 380 |
| 81 | BA | 5C8 | 340 |
| 82 | BA | 5C8 | 300 |
| 83 | BA | 5C8 | 380 |

**Table 4**

| Effects of High and Low Dose 5D12 | | | |
|---|---|---|---|
| **Graft number** | **Donor** | **Treatment** | **Epidermal thickness (mm)** |
| 84 | TR | Isotype control | 420 |
| 85 | TR | Isotype control | 300 |
| 86 | TR | Isotype control | 320 |
| 87 | TR | Low dose 5D12 | 340 |
| 88 | TR | Low dose 5D12 | 300 |
| 89 | TR | Low dose 5D12 | 300 |
| 90 | TR | High dose 5D12 | 250 |
| 91 | TR | High dose 5D12 | 270 |
| 92 | TR | High dose 5D12 | 210 |
| 93 | TR | 5C8 | 290 |
| 94 | TR | 5C8 | 250 |
| 95 | TR | 5C8 | 300 |

### EXAMPLE 4

### Evaluation of Methotrexate Treatment in Combination with 5D12

The effects of methotrexate treatment alone or in combination with low dose (0.5 mg/kg) 5D12 treatment were evaluated. An increasing dosing regimen was chosen, starting with 0.1 mg/kg and increasing the dose by 0.05 mg/kg every week until 0.4 mg/kg was reached. The scheme was combined with the application of the isotype control antibody or with the low dose 5D12 treatment schedule. The isotype control treatment served as a negative control and the low dose 5D12 treatment as a positive control.

Methotrexate in combination with the isotype control antibody had only minimal effecs on epidermal thickness, and normalization of the histological characteristics could not be observed (Tables 5-7). Thus, methotrexate alone given according to this schedule does not represent a suitable regimen for the therapy of lesional psoriatic skin grafted onto SCID mice.

When combined with low dose 5D12, the changes induced in comparison with the negative control were similar to those seen in the positive control group receiving low dose 5D12 only (Tables 5-7).

**Table 5**

| Effects of Low Dose 5D12 in Combination with Methotrexate | | | |
|---|---|---|---|
| **Graft number** | **Donor** | **Treatment** | **Epidermal thickness (mm)** |
| 140 | KO | Isotype control | 340 |
| 141 | KO | Isotype control | 300 |
| 142 | KO | Isotype control | 320 |
| 143 | KO | Low dose 5D12 | 270 |
| 144 | KO | Low dose 5D12 | 210 |
| 145 | KO | Low dose 5D12 | 210 |
| 146 | KO | MTX plus isotype | 300 |
| 147 | KO | MTX plus isotype | 250 |
| 148 | KO | MTX plus isotype | 270 |
| 149 | KO | MTX plus 5D12 | 190 |
| 150 | KO | MTX plus 5D12 | 250 |
| 151 | KO | MTX plus 5D12 | 210 |

**Table 6**

| Effects of Low Dose 5D12 in Combination with Methotrexate | | | |
|---|---|---|---|
| **Graft number** | **Donor** | **Treatment** | **Epidermal thickness (mm)** |
| 152 | ME | Isotype control | 460 |
| 153 | ME | Isotype control | 380 |
| 154 | ME | Isotype control | 380 |
| 155 | ME | Low dose 5D12 | 380 |
| 156 | ME | Low dose 5D12 | 300 |
| 157 | ME | Low dose 5D12 | 320 |
| 158 | ME | MTX plus isotype | 340 |
| 159 | ME | MTX plus isotype | 320 |
| 160 | ME | MTX plus isotype | 380 |
| 161 | ME | MTX plus 5D12 | 340 |
| 162 | ME | MTX plus 5D12 | 320 |
| 163 | ME | MTX plus 5D12 | 290 |

**Table 7**

| Effects of Low Dose 5D12 in Combination with Methotrexate | | | |
|---|---|---|---|
| **Graft number** | **Donor** | **Treatment** | **Epidermal thickness (mm)** |
| 164 | HU | Isotype control | 380 |
| 165 | HU | Isotype control | 420 |
| 166 | HU | Isotype control | 380 |
| 167 | HU | Low dose 5D12 | 250 |
| 168 | HU | Low dose 5D12 | 210 |
| 169 | HU | Low dose 5D12 | 270 |
| 170 | HU | MTX plus isotype | 290 |
| 171 | HU | MTX plus isotype | 240 |
| 172 | HU | MTX plus isotype | 340 |
| 173 | HU | MTX plus 5D12 | 210 |
| 174 | HU | MTX plus 5D12 | 190 |
| 175 | HU | MTX plus 5D12 | 250 |

As shown in Tables 5-7, combination of 5D12 with methotrexate failed to induce changes superior to 5D12 alone. The changes seen in the 5D12-treated grafts again confirmed that this antibody is an effective treatment modality. Although doses as low as 0.5 mg/kg of antibody were effective, higher doses, such as 1 mg/kg, 1.2 mg/kg, 1.4 mg/kg, 1.6 mg/kg, 1.8 mg/kg, 2.0 mg/kg, 2.1 mg/kg, 2.2 mg/kg, 2.3 mg/kg, 2.4 mg/kg, or 2.5 mg/kg may be preferable, to avoid the potential of too low dosing.

From the foregoing it will be appreciated that, although specific embodiments of the invention have been described herein for purposes of illustration, various modifications may be made without deviating from the spirit and scope of the invention. Accordingly, the invention is not to be limited except as by the appended claims.

## Claims

1. A composition for treating an autoimmune disease, comprising a therapeutically effective amount of a CD40 antagonist in a pharmaceutically acceptable carrier.

2. The composition of claim 1, wherein said CD40 antagonist is small molecule.

3. The composition of claim 1, wherein said CD40 antagonist is a protein.

4. The composition of claim 1, wherein said CD40 antagonist is an antibody.

5. The composition of claim 1, wherein said CD40 antagonist is a monoclonal antibody.

6. The composition of claim 1, wherein said CD40 antagonist is a humanized monoclonal antibody.

7. The composition of claim 1, wherein said CD40 antagonist is a human monoclonal antibody.

8. The composition of claim 1, wherein said CD40 antagonist is antibody 5H7.

9. The composition of claim 1, wherein said autoimmune disease is psoriasis.

10. A method of treating a mammal afflicted with an autoimmune disease, comprising administering to said mammal a therapeutically effective amount of a CD40 antagonist thereby reducing the severity of said autoimmune disease.

11. The method of claim 10, wherein said antagonist blocks the interaction between CD40 and CD40L.

12. The method of claim 10, wherein said antagonist is an antibody that binds to CD40.

13. The method of claim 10, wherein said antibody is a monoclonal antibody.

14. The method of claim 10, wherein said antibody is a humanized monoclonal antibody.

15. The method of claim 10, wherein said antibody is a human monoclonal antibody.

16. The method of claim 10, wherein said antibody is 5H7.

17. The method of claim 10, wherein said antagonist is a fragment of antibody 5H7.

18. The method of claim 10, wherein said autoimmune disease is a T-cell-mediated inflammatory skin disease.

19. The method of claim 10, wherein said autoimmune disease is psoriasis.

20. The method of claim 10, wherein said mammal is human.

21. The method of claim 10, wherein said CD40 antagonist is administered intraperitoneally or intradermally.

22. A method of treating a mammal afflicted with a neoplastic disease, comprising administering to said mammal a therapeutically effective amount of a CD40 antagonist thereby reducing the severity of said neoplastic disease.

23. A method of treating a mammal afflicted with psoriasis, comprising administering to said mammal a therapeutically effective amount of a CD40 antagonist.
